Europäisches Patentamt

European Patent Office

Office européen des brevets

(11) Veröffentlichungsnummer : **0 012 304**
**B1**

(12) **EUROPÄISCHE PATENTSCHRIFT**

(45) Veröffentlichungstag der Patentschrift :
03.02.82

(21) Anmeldenummer : 79104818.4

(22) Anmeldetag : 01.12.79

(51) Int. Cl.³ : **C 07 D323/06**

(54) **Verfahren zur kontinuierlichen Herstellung von Trioxan.**

(30) Priorität : 08.12.78 DE 2853091

(43) Veröffentlichungstag der Anmeldung :
25.06.80 (Patentblatt 80/13)

(45) Bekanntmachung des Hinweises auf die Patenterteilung : 03.02.82 Patentblatt 82/05

(84) Benannte Vertragsstaaten :
AT BE CH DE FR GB IT NL SE

(56) Entgegenhaltungen :
DE - A - 1 543 390
DE - A - 2 428 719
FR - A - 1 429 161
GB - A - 1 012 372

(73) Patentinhaber : HOECHST AKTIENGESELLSCHAFT
Postfach 80 03 20
D-6230 Frankfurt/Main 80 (DE)

(72) Erfinder : Mück, Karl-Friedrich, Dr.
Wittenberger Strasse 17
D-6200 Wiesbaden (DE)
Erfinder : Sextro, Günter, Dr.
Erbsenacker 43
D-6200 Wiesbaden (DE)
Erfinder : Burg, Karlheinz, Dr.
Eichenweg 18
D-6200 Wiesbaden (DE)

Imprimerie Jouve, 18, rue St-Denis, 75001 Paris, France

# Verfahren zur kontinuierlichen Herstellung von Trioxan

Die Herstellung von Trioxan aus wässrigem Formaldehyd ist verschiedentlich in der Literatur beschrieben (vgl. Walker, Formaldehyde, Reinhold Publ. New York, 3. Auflage, 1964, Seite 198-199). Das bei höheren Temperaturen in Gegenwart saurer Katalysatoren gebildete Trioxan wird hierbei durch Destillation aus dem Reaktionsgemisch abgetrennt. Der Synthesedampf, der außer Trioxan Wasser und Formaldehyd noch Nebenprodukte der Synthese enthält, wird meist entsprechend der US-Patentschrift 2 304 080 in einer dem Reaktor aufgesetzten Verstärkerkolonne oder entsprechend der GB-PS 1 012 372 in einer mit Verstärker und Abtriebsteil versehenen Kolonne rektifiziert. Die erhaltene trioxanreiche Fraktion wird durch Extraktion und/oder ein anderes bekanntes Trennverfahren weiter aufgearbeitet.

Es ist bekannt, daß die Zeitausbeuten (g Trioxan pro kg Formaldehyd und Stunde) bei der Trioxansynthese gering sind. Nach der DE-PS 1 135 491 werden z.B. durch einfache Destillation des Reaktionsgemisches aus dem Reaktionsgefäß Zeitausbeuten von 152 g Trioxan pro kg Formaldehyd und Stunde erreicht. Die geringen Zeitausbeuten haben zur Folge, daß bei der Herstellung von Trioxan aus wässrigen Formaldehyd-Lösungen lange Verweilzeiten benötigt werden. Außerdem sind auch große Reaktionsvolumina erforderlich, um technisch zufriedenstellende Raumzeitausbeuten (g Trioxan pro l Reaktionsvolumen und Zeit) zu erhalten.

Zur Erhöhung der Raumzeitausbeuten bei der Trioxansynthese wurde bereits vorgeschlagen, das chemische Gleichgewicht zwischen Formaldehyd und Trioxan im Reaktionsgemisch möglichst weitgehend durch Arbeiten bei hohen Verdampfungsgeschwindigkeiten zu stören.

Diese Verfahrensweise führt jedoch zu niedrigen Trioxankonzentrationen im Synthesedampf (vgl. E. Bartholomé, Chem. Ing. Techn. 43 (1971) 597). Die Aufarbeitung solcher Reaktionsdämpfe ist wegen der niedrigen Trioxankonzentration sehr energieintensiv.

Weiterhin ist aus der DE-AS 1 543 390 ein Verfahren bekannt geworden, für das als maximaler Wert der Zeitausbeute 1090 g Trioxan pro kg Formaldehyd und Stunde genannt werden. Nach diesen Verfahren wird wässrige Formaldehyd-Lösung wie üblich in Gegenwart von sauren Katalysatoren in einem Umlaufverdampfer zum Sieden erhitzt, und die trioxanreichen Synthesedämpfe werden dann über eine, dem Reaktor aufgesetzte Kolonne entfernt. In diese Kolonne wird dem Synthesedampf Reaktionsflüssigkeit entgegengeleitet, die sich im chemischen Gleichgewicht befindet. Hierdurch wird erreicht, daß die Trioxankonzentration der Synthesedämpfe auch bei hohen Verdampfungsgeschwindigkeiten den Gleichgewichtswert erreicht, der sonst nur bei niedrigen Verdampfungsgeschwindigkeiten erhalten wird und der dem Verteilungsgleichgewicht von Trioxan in gasförmiger und flüssiger Phase entspricht.

Nachteil des Verfahrens gemäß dieser DE-AS 1 543 390 ist jedoch, daß Zusatzaggregate benötigt werden, die eine erhebliche Vergrößerung des eigentlichen Reaktorvolumens und damit eine Erniedrigung der Raumzeitausbeuten bedeuten. So besteht in dem beschriebenen Verfahren der mit der Reaktionsflüssigkeit in Kontakt kommende Verfahrensteil aus dem eigentlichen Reaktor, einem Verdampfer, einer Pumpe, einer langen Rohrleitung, gegebenenfalls mit Verweilgefäß, und einer Kolonne. All diese Teile müssen aus Materialien gefertigt sein, die gegenüber einer etwa 100 °C heißen sauren, z.B. schwefelsauren Formaldehyd-Lösung beständig sind. Die generellen Nachteile von korrosionsbeständigen Reaktoren sind in der DE-AS 2 103 687 beschrieben. Berechnet man nun die Raumzeitausbeuten, dann ergeben sich wesentlich geringere Werte als für die in der DE-AS 1 543 390 angegebenen Zeitausbeuten.

Schließlich wird in der DE-OS 2 428 719 noch ein Verfahren zur Abtrennung von Trioxan aus wäßrigen Lösungen, die Trioxan zusammen mit Formaldehyd enthalten, beschrieben, wonach 5 bis 15 Gew.-% der Lösungen bei Temperaturen unter 100 °C unter vermindertem Druck und bei Verweilzeiten von weniger als 1 Minute abdestilliert werden und anschließend das Trioxan aus dem Destillat isoliert wird. Nachteilig an dieser Verfahrensweise sind die geringen Zeitausbeuten.

Aufgabe der Erfindung war es daher, ein Verfahren zur Herstellung von Trioxan, ggfs. zusammen mit cyclischen Formalen, bereitzustellen, bei dem die Nachteile des Standes der Technik vermieden oder zumindestens beträchtlich vermindert werden.

Erfindungsgemäß wird dies durch eine Verfahrensweise erreicht, bei der in einem Umlaufreaktor mit Verdampfer bestimmte Mengen des umlaufenden Reaktionsgemisches verdampft werden.

Die vorliegende Erfindung betrifft daher ein Verfahren zur kontinuierlichen Herstellung von Trioxan aus wäßrigen Formaldehyd-Lösungen in Gegenwart saurer Katalysatoren in einem Umlaufreaktor mit Verdampfer bei Verweilzeiten zwischen 2 und 240 Minuten, das dadurch gekennzeichnet ist, daß die das System verlassende Dampfmenge, bezogen auf die insgesamt umgepumpte Produktmenge, 0,1 bis 4 % beträgt.

Weiterhin hat die Erfindung ein kontinuierliches Verfahren zur gleichzeitigen Herstellung von Trioxan und cyclischen Formalen zum Gegenstand, wobei eine wäßrige Formaldehyd-Lösung, die mindestens ein Diol und/oder mindestens ein Epoxyd enthält, in der vorstehenden Weise behandelt wird.

Nach dem erfindungsgemäßen Verfahren werden in überraschender Weise Trioxankonzentrationen im Synthesedampf auch bei hohen Verdampfungsgeschwindigkeiten von 1,5 bis 3,0 kg Reaktionsdampf pro Stunde und pro kg Reaktionsmischung erhalten, die den maximal erreichbaren Gleichgewichtswerten entsprechen.

Die Trimerisierungsreaktion von Formaldehyd zur Trioxanherstellung erfolgt in an sich bekannter Weise durch Umsatz von wäßrigen, im allgemeinen 30-80 %igen, vorzugsweise 40-70 %igen Formaldehyd-Lösungen, gegebenenfalls unter Zusatz der bekannten Antischaummittel, in Gegenwart der hierfür bekannten sauren Katalysatoren, wie Mineralsäuren, starken organischen Säuren oder einer in seiner katalytischen Aktivität entsprechenden Menge eines anderen sauren Katalysators. Als saure Katalysatoren, die weniger flüchtig als das Reaktionsgemisch sein müssen, haben sich beispielsweise Schwefelsäure, Phosphorsäure, p-Toluolsulfonsäure oder saure Ionenaustauscher als brauchbar erwiesen. Die Menge ist nicht kritisch und beträgt in der Regel 2 bis 25 %, vorzugsweise 2 bis 10 %.

Falls nach der erfindungsgemäßen Variante ein Gemisch aus Trioxan und mindestens einem cyclischen Formal hergestellt werden soll, werden der wäßrigen Formaldehydlösung zweckmäßigerweise 1 bis 25 Gew.-%, vorzugsweise 2 bis 15 Gew.-%, bezogen auf Formaldehyd, an mindestens einem Diol und/oder mindestens einem Epoxyd zugesetzt.

Die hierfür in Frage kommenden Diole sind vor allem 1,2 Diole, 1,3-Diole und α,ω-Diole. Ebenso können statt der 1,2-Diole auch die entsprechenden Epoxyde oder Gemische aus beiden eingesetzt werden. Vorzugsweise werden Diole verwendet, deren cyclische Formale Siedepunkte von kleiner als 150 °C haben und/oder mit Wasser niedrigsiedende Azeotrope ( < 150 °C) bilden oder wasserdampfflüchtig sind. Als geeignet haben sich z.B. Äthylenglykol, Äthylenoxyd, Propylenglykol-1,2, Propylenoxyd, Propylenglykol-1,3, Butandiol-1,2, Butandiol-1,3, Butandiol-1,4 und Buten(3)-diol-1,2 erwiesen. Vorzugsweise werden erfindungsgemäß dabei Äthylenglykol bzw. Äthylenoxyd, Propylenglykol-1,2 und Butandiol-1,4 eingesetzt und besonders bevorzugt Äthylenglykol bzw. Äthylenoxyd.

Die Reaktion wird erfindungsgemäß in einem bekannten Umlaufreaktor mit Verdampfer durchgeführt. Dafür eignen sich beispielsweise Zwangsumlaufverdampfer, Fallfilmverdampfer oder Dünnschichtverdampfer mit Zwangsumlauf. Derartige Systeme sind zum Beispiel in Ullmann, Bd. 1 (1951), 3. Auflage. Seiten 533-537 beschrieben. Besonders geeignet sind Zwangsumlaufverdampfer.

Die Verweilzeit der Reaktionsmischung im Reaktorsystem beträgt 2 bis 240 Minuten, vorzugsweise 5 bis 120 Minuten und besonders bevorzugt 15 bis 60 Minuten. Die Temperaturen der Reaktionsmischung liegen je nach Druck zwischen 50 °C und 150 °C, vorzugsweise 95 °C und 110 °C.

Das Reaktionsprodukt bestehend aus Trioxan, Formaldehyd und Wasser sowie ggf. cyclischen Formalen wird mit Hilfe des Verdampfers destillativ aus dem Reaktorsystem entfernt. Hierzu kann bei Normaldruck, unter vermindertem Druck, beispielsweise zwischen 150 und 950 mbar oder unter Überdruck, beispielsweise 1-4 bar, gearbeitet werden. Vorzugsweise wird bei Normaldruck gearbeitet.

Entsprechend dem erfindungsgemäßen Verfahren beträgt die das System verlassende Destillatmenge, bezogen auf die durch den Verdampfer umlaufende Produktmenge, 0,1 bis 4,0 %. Vorzugsweise liegt dieser Bereich bei 0,2 bis 3,0 % und besonders bevorzugt ist der Bereich von 0,5 bis 2,0 %. Dieser geforderte Verdampfungsanteil kann z.B. durch die Pumpe eines Zwangsumlaufverdampfers sichergestellt werden. Die Förderleistung der Pumpe muß so bemessen sein, daß sie dem 1 000 bis 25-fachen, vorzugsweise dem 500 bis 33-fachen und besonders vorteilhaft dem 200 bis 50-fachen der das System pro Stunde verlassenden Produktmenge entspricht. Nach einer weiteren Ausführungsform kann ein Teilstrom des umlaufenden Gemisches vor Eintritt in den Verdampfer abgezweigt und mit dem, den Verdampfer verlassenden Produktstrom vor Eintritt in den Dampfraum des Reaktionskessels vereinigt werden. Hiermit können die Strömungsgeschwindigkeiten im Verdampfer herabgesetzt werden. Die Menge des Teilstromes muß dabei so bemessen sein, daß die das System verlassende Produktmenge, bezogen auf die insgesamt umgepumpte Produktmenge, 0,1 bis 4 % beträgt. Beispielsweise kann die Menge des Teilstromes bis zum Doppelten der den Verdampfer durchlaufenden Produktmenge betragen.

Der das Reaktorsystem verlassende Synthesedampf wird in üblicher Weise entweder als Dampf oder als Kondensat mittels einer Rektifikation, wie z.B. in der GB-PS 1 012 372 beschrieben, angereichert. Die anfallende trioxanreiche Fraktion, die ggf. auch cyclische Formale enthält, kann dann z.B. durch Extraktion mit einem mit Wasser nichtmischbaren Lösungsmittel für Trioxan und ggf. cyclische Formale, wie Methylenchlorid und anschließender Neutralisation und fraktionierte Destillation oder Kristallisation gereinigt werden. Auch andere bekannte Trennverfahren können hierfür Einsatz finden, wie beispielsweise in Process Economics Program, Stanford Institut Report 23 (1967) S. 181 oder in der DE-OS 1 570 335 beschrieben.

Das erfindungsgemäße Verfahren ist auch verfahrenstechnisch besonders vorteilhaft, da hierbei außer z.B. einer zwischen Reaktor und Verdampfer installierten Pumpe keine Vergrößerung des Reaktionsraums durch Zusatzaggregate notwendig ist, um auch bei hohen Verdampfungsgeschwindigkeiten Synthesedampf mit dem höchstmöglichen Trioxangehalt, der dem Gleichgewichtswert in der Gasphase für das Verteilungsgleichgewicht von Trioxan in flüssiger und gasförmiger Phase entspricht, zu erzeugen. Nach früher beschriebenen Verfahren ist dieser Trioxangehalt nur bei Arbeiten mit niederen Verdampfungsgeschwindigkeiten oder nach der in der DE-AS 1 543 390 beschriebenen, aufwendigeren Verfahrensweise realisierbar. Erfindungsgemäß sind somit Raumzeitausbeuten erreichbar, die wesentlich höher sind, als die nach den früher beschriebenen Verfahren erzielten.

Das erfindungsgemäße Verfahren ermöglicht außerdem eine Trioxansynthese mit einem minimalen Energiebedarf ; es weist also eine günstige Energiebilanz auf.

Weiterhin zeichnet sich die Verfahrensweise gemäß der Erfindung dadurch aus, daß durch die kurzen Verweilzeiten bei Anwendung hoher Verdampfungsgeschwindigkeiten, die Bildung von Nebenprodukten wie z.B. Ameisensäure zurückgedrängt wird. In gleicher Weise wirken sich geringe Katalysatorsäure-Konzentrationen oder die Verwendung von Ionenaustauscher aus.

Die folgenden Beispiele sollen das erfindungsgemäße Verfahren näher erläutern.

## Beispiele

Die verwendete Versuchsapparatur ist in anliegender Figur skizziert. Der Reaktor besteht daher aus dem Kessel (1), dem Verdampfer (2), im speziellen Fall einem Röhrenwärmetauscher und der Pumpe (3). Bei (4) wird wäßrige Formaldehydlösung nachdosiert. Der saure Katalysator wird zusammen mit wässriger Formaldehydlösung im Reaktionsgefäß (1) vorgelegt, während bei (5) das Destillat abgezogen wird.

Im Reaktionskessel (1) werden jeweils 700 g einer Mischung (400 g in den Vergleichsversuchen 4 und 5) bestehend aus 90 Teilen einer 63,5 %igen wässrigen Formaldehydlösung und 10 Teilen konzentrierter Schwefelsäure vorgelegt. Der Kessel (1) ist auf 95 °C temperiert. Das Gemisch wird mittels der Pumpe (3) durch den Verdampfer (2) gepumpt. Der Verdampfer (2) wird je nach dem gewünschten Durchsatz verschieden stark beheizt. Der verdampfte Anteil des umgepumpten Reaktionsgemisches wird in einem Quenchsystem total kondensiert. Das Kondensat wird auf Trioxan und Formaldehydgehalt untersucht. Entsprechend dem verdampften Anteil wird 63,5 %ige Formaldehydlösung nachdosiert. Die Versuchsdauer betrug jeweils 5 Stunden. Die Ergebnisse sind zusammen mit den Vergleichsversuchen in Tabelle 1 und Tabelle 2 zusammengestellt.

Die Beispiele 1 bis 3 (Tabelle 1) zeigen, daß die Trioxankonzentrationen im Synthesedampf unabhängig vom Durchsatz sind, wenn der Verdampfungsanteil, d.h. der das System verlassende Dampf bezogen auf die durch den Verdampfer umlaufende Produktmenge, kleiner 4 % ist, während bei hohen Verdampfungsanteilen (10 %) der Trioxangehalt im Reaktionsdampf stark mit steigendem Druchsatz abnimmt (Vergleichsversuche 4 und 5).

Vergleicht man das Beispiel Nr. 3 mit Vergleichsversuch 6 (entsprechend dem Beispiel Nr. 2 der DE-AS 1 543 390), so wird deutlich, daß Verweilzeit und Trioxankonzentrationen im Synthesedampf zwar identisch sind, daß sich jedoch durch das wesentlich gering··re Reaktionsvolumen des erfindungsgemäßen Verfahrens beträchtlich höhere Raumzeitausbeuten ergeben (siehe Tabelle 2).

## Tabelle 1

| Beispiel Nr. | Durchsatz kg/kg.h | Trioxan im Destillat % | Verdampfungs- anteil % | Umsatz % | Verweilzeit h |
|---|---|---|---|---|---|
| 1 | 1,07 | 20,6 | 0,93 | 32,4 | 0,94 |
| 2 | 1,67 | 20,5 | 1,5 | 32,3 | 0,6 |
| 3 | 2,91 | 20,4 | 1,3 | 32,1 | 0,3 |
| 4* | 0,42 | 20,3 | 10,0 | 32,0 | 1,4 |
| 5* | 0,97 | 8,7 | 10,0 | 13,7 | 0,6 |

* Vergleichsversuche

## Tabelle 2

| Beispiel Nr. | Formaldehyd- Konzentration Einlauf % | Verweilzeit h | Trioxan im Destillat % | Reaktorvolumen | Verhältnis der Reaktions- volumina | Umsatz zu Trioxan % | Verhältnis der Raumzeit-Ausbeuten |
|---|---|---|---|---|---|---|---|
| 3 | 63,5 | 0,3 | 20,4 | Reaktor, Verdampfer, Pumpe | 1 | 32,1 | 1 |
| 6* | 63,5 | 0,3 | 20,8 | Reaktor, Pumpe Verdampfer, Kolonne, lange Rohrleitung | 1,5-2 | 32,8 | 0,68-0,51 |

* gemäß Beispiel 2 der DE-AS 1.543.390

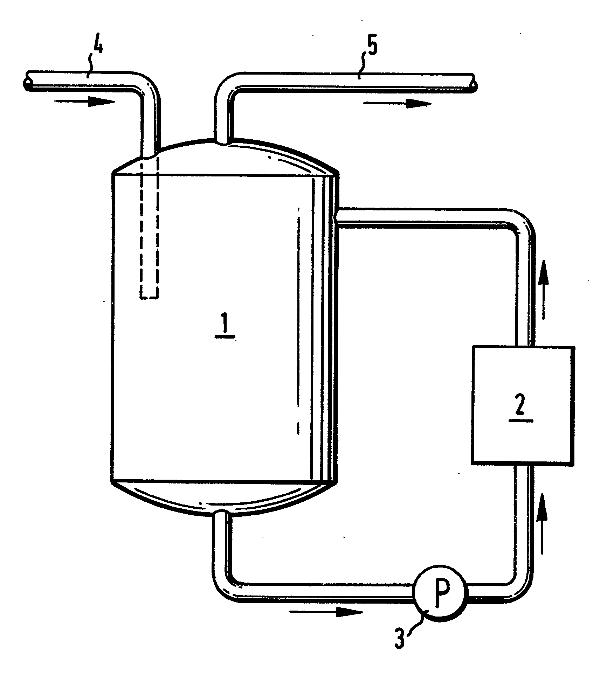

## Ansprüche

1. Verfahren zur kontinuierlichen Herstellung von Trioxan gegebenenfalls zusammen mit cyclischen Formalen, aus wäßrigen Formaldehyd-Lösungen, die gegebenenfalls mindestens ein Diol und/oder mindestens ein Epoxyd enthalten, in Gegenwart von sauren Katalysatoren in einem Umlaufreaktor mit Verdampfer bei Verweilzeiten von 2 bis 240 Minuten, dadurch gekennzeichnet, daß die das System verlassende Dampfmenge, bezogen auf die insgesamt umgepumpte Produktmenge, 0.1 bis 4 % beträgt.

2. Verfahren gemäß Anspruch 1, dadurch gekennzeichnet, daß ein Zwangsumlaufverdampfer verwendet wird.

3. Verfahren gemäß Anspruch 1 und 2, dadurch gekennzeichnet, daß die das System verlassende Dampfmenge, bezogen auf die insgesamt umgepumpte Produktmenge, 0,2 bis 3 % beträgt.

4. Verfahren gemäß Anspruch 1 bis 3, dadurch gekennzeichnet, daß ein Teilstrom des umlaufenden Gemisches vor Eintritt in den Verdampfer abgezweigt und mit dem, den Verdampfer verlassenden Produktstrom vor Eintritt in den Dampfraum des Reaktors wieder vereinigt wird.

## Claims

1. A process for the continuous manufacture of trioxane, optionally together with cyclic formals, from aqueous formaldehyde solutions containing optionally at least one diol and/or at least one epoxide, in the presence of acidic catalysts, in a circulation reactor with evaporator, at a residence time in the range of from 2 to 240 minutes, wherein the amount of vapor leaving the system is from 0.1 to 4 % relative to the total amount of circulating product.

2. The process as claimed in claim 1, which comprises using a forced circulation evaporator.

3. The process as claimed in claims 1 and 2, wherein the vapor amount leaving the system is in the range of from 0.2 to 3 %, relative to the total amount of circulating product.

4. The process as claimed in claims 1 to 3, which comprises deriving a partial current of the circulating mixture before entry into the evaporator and uniting it before entry into the vapor zone of the reactor with the product current leaving the evaporator.

## Revendications

1. Procédé pour préparer en continu du trioxanne, éventuellement accompagné d'autres formals cycliques, à partir de solutions aqueuses de formaldéhyde renfermant éventuellement au moins un diol et/ou un époxyde, en présence de catalyseurs acides, dans un réacteur à circulation équipé d'un évaporateur, avec des temps de séjour de 2 à 240 minutes, procédé caractérisé en ce que la quantité de vapeur sortant du système, par rapport à la quantité totale de produit recyclé par pompage, est de 0,1 à 4 %.

2. Procédé selon la revendication 1, caractérisé en ce qu'on utilise un évaporateur à circulation forcée.

3. Procédé selon l'une des revendications 1 et 2, caractérisé en ce que la quantité de vapeur sortant du système, par rapport à la quantité totale de produit recyclé par pompage, est de 0,2 à 3 %.

4. Procédé selon l'une quelconque des revendications 1 à 3, caractérisé en ce qu'un courant partiel du mélange en circulation est dérivé avant son entrée dans l'évaporateur et est à nouveau réuni, avant son entrée dans la phase vapeur du réacteur, avec le courant de produit sortant de l'évaporateur.